# EUROPÄISCHE PATENTSCHRIFT

(11) **EP 0 916 306 B1**
(45) Veröffentlichungstag und Bekanntmachung des Hinweises auf die Patenterteilung: **31.10.2001**
(21) Anmeldenummer: 98810895.7
(22) Anmeldetag: 09.09.1998
(51) Int. Cl.: A61B 3/135

(54) **Vorrichtung zur stereoskopischen Untersuchung eines Patientenauges**
Apparatus for stereoscopic examination of a patient's eye
Appareil destiné à l'examen stéréoscopique de l'oeil d'un patient

(30) Priorität: 11.11.1997 EP 97810857; 29.04.1998 EP 98810380
(43) Veröffentlichungstag der Anmeldung: 19.05.1999
(73) Patentinhaber: HAAG-STREIT AG, CH-3098 Köniz (CH)
(72) Erfinder: Lobel, David, Dr., Afeka, Tel-Aviv (IL); Ulbers, Gerd, Dr., 3132 Riggisberg (CH); Widmer, Hansruedi, 3145 Niederscherli (CH); Fankhauser, Hans, 3255 Rapperswil (CH); Pertz, Eberhard, 1066 Epalinges (CH); Studer, Reto, 1580 Avenches (CH)
(74) Vertreter: Roshardt, Werner Alfred, Dipl.-Phys.

(56) Entgegenhaltungen:
- EP-A- 0 091 334
- EP-A- 0 712 600
- DE-B- 1 133 911
- DE-U- 9 308 464
- DE-U- 29 514 224
- US-A- 4 331 392
- US-A- 5 216 456

## Beschreibung

### Technisches Gebiet

Die Erfindung betrifft eine Vorrichtung gemäss dem Oberbegriff des Patentanspruchs 1 sowie eine Linsentrageinheit als Zusatzteil für die Vorrichtung.

### Stand der Technik

Ein Spaltlampengerät ist beispielsweise von der Firma Haag-Streit unter der Bezeichnung "Orginal-Spaltlampe 900 BM" bekannt und ist mit seinem wesentlichen Merkmalen in der DE-A 1 133 911 beschrieben. Das bekannte Spaltlampengerät hatte zur stereoskopischen Betrachtung eines Auges eine Betrachtungseinheit sowie eine Beleuchtungseinheit für das zu betrachtende Auge. Der Querschnitt eines Beleuchtungsfleckes auf bzw. im Auge ist durch eine in der Breiten- und Höhenabmessung verstellbare Blende einstellbar. Die Beleuchtungseinheit war auf einem vertikal verlaufenden Ast einer Halteeinheit angeordnet. Das zu betrachtende Auge war in einer annähernd horizontal verlaufenden Ebene auf einer Seite der Halteeinheit positionierbar. Die Betrachtungseinheit war annähernd in der Ebene auf der hierzu gegenüberliegenden Seite der Halteeinheit angeordnet. Die Halteeinheit hatte drei Säulen. Auf den beiden äusseren Säulen ruhte die Beleuchtungsoptik. Auf der dritten, mittleren, als Stummelsäule ausgebildeten Säule war ein Umlenkspiegel angeordnet, der den Strahl der Beleuchtungseinheit zum Auge lenkte. In den Zwischenräumen zwischen jeweils einer äusseren Säule und der Stummelsäule wurden die Strahlengänge zur Betrachtungseinheit geführt.

In der US-A 5 216 456 ist ein dreisäuliges Spaltlampengerät beschrieben, wobei die mittlere Säule den Umlenkspiegel für die Augenbeleuchtung trägt. Alle drei Säulen werden über eine Verbindungsplatte zusammengefaßt, auf der dann eine Beleuchtungseinheit aufgesetzt ist.

Bei der US-A 4 331 392 ist die Beleuchtungseinheit im unteren Teil des Spaltlampengeräts angeordnet und hat somit zwangsläufige eine sich vollständig von der Erfindung unterscheidende Konstruktion, bei der die Beleuchtungseinheit oben angeordnet ist. Die Anordnung einer Beleuchtungseinheit im oberen Teil des Spaltlampengeräts gestattet gegenüber der Anordnung der US-A 4 331 392 ein einfaches Auswechseln der Beleuchtungsquelle. Das Spaltlampengerät der US-A 4 331 392 ist gattungsfremd zu dem der Erfindung und wird somit nachfolgend nicht weiter betrachtet.

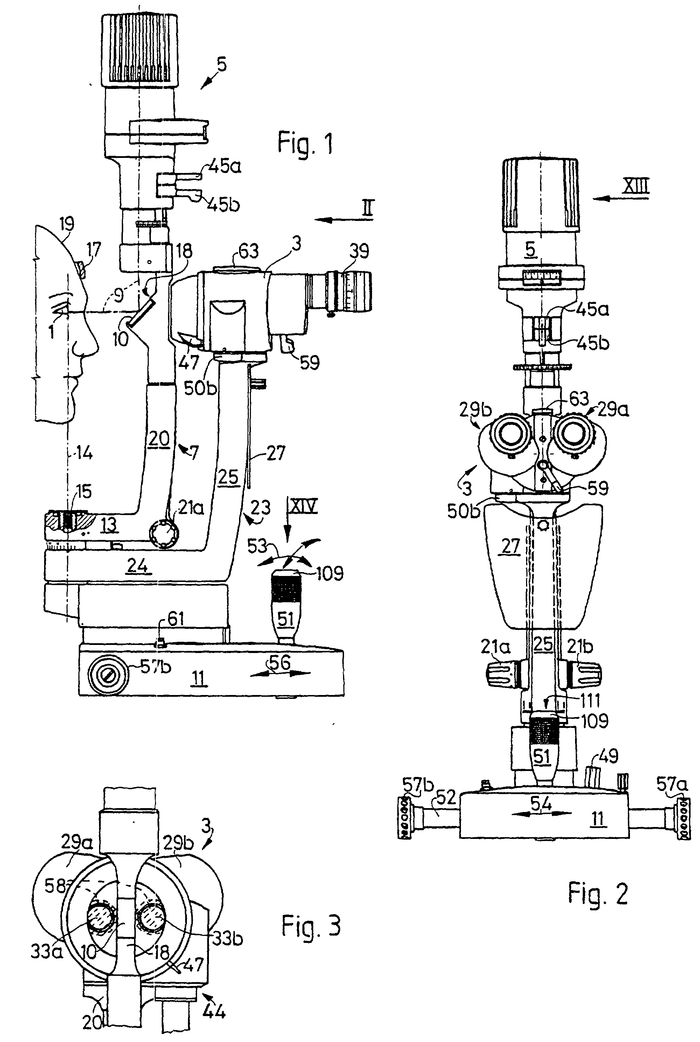

In der EP-A 0 091 334 ist ein Spaltlampengerät beschrieben, mit dem die Augenbetrachung möglich war und ein Laserstrahl zur Augenbehandlung geführt wurde. Das Spaltlampengerät der EP-A 0 091 334 war analog zu demjenigen der DE-A 1 133 911 aufgebaut, wobei für die Laserstrahlführung ein zusätzlicher Säulenstumpf vorhanden ist. Der analoge Aufbau ist insbesondere in der Figur 2, welche einen vertikalen Längsschnitt durch das Gerät darstellt zu sehen. In Figur 2 ist geschnitten die den Umlenkspiegel tragende mittlere Säule zu sehen. Femer ist vom Blickfeld des Patienten aus die linke seitliche Säule dargestellt. Die Verbindungsplatte für die beiden seitlichen Säulen, auf der die Beleuchtungseinheit (hier mit 20 gekennzeichnet) sitzt, ist geschnitten dargestellt.

### Darstellung der Erfindung

### Aufgabe der Erfindung

Aufgabe der Erfindung ist es, eine Vorrichtung, insbesondere ein sog. Spaltlampengerät zu schaffen, welche bzw. welches einen guten Patienten-Arzt-Kontakt erlaubt, eine effiziente Untersuchung gewährleistet und sich bei einer ästhetisch ansprechenden Formgestaltung mit ausgezeichneten optischen Eigenschaften preislich günstig herstellen lässt.

### Lösung der Aufgabe

Die Lösung der Aufgabe ist Gegenstand des Patentanspruchs 1.

Erst durch einen erfindungsgemässen konstruktiven Aufbau, der sich durch möglichst wenig Material zwischen dem beobachtenden und untersuchenden Arzt sowie dem Patienten auszeichnet, ist die effiziente Untersuchung und der gute Patienten-Arzt-Kontakt gewährleistet, der auch noch durch die hierbei erreichte ästhetisch vollendete Formgebung unterstützt wird. Eine materialvermeidende Konstruktion zwischen Arzt und Patienten wird erfindungsgemäss dadurch erreicht, dass der vertikal verlaufende Ast einer Halteeinheit für eine Beleuchtungseinheit einsäulig mit einem schmalen Säulenquerschnitt ausgebildet ist. Der schmale Säulenbereich wird bevorzugt in Augenhöhe ausgebildet. Die effiziente Untersuchung wird femer dadurch unterstützt, daß gemäss den Merkmalen des Patentanspruchs 6 eine Videoaufzeichnungsmöglichkeit gegeben ist. Durch die hier erreichte kompakte Gestaltung ist ebenfalls eine preisgünstige Herstellung möglich. Auch durch die Merkmale des Patentanspruchs 8 wird die Untersuchungseffizienz gesteigert, da die betrachtende, beobachtende bzw. untersuchende Person ihren Blick nicht mehr von der Betrachtungseinheit zum Suchen von Bedienelementen wenden muss. Die wichtigsten Bedienelemente können jetzt lediglich mit einer einzigen Hand bedient werden.

Wird nun als Beobachtungseinheit bevorzugt im Zusammenhang mit der schlanken Haltesäule als Halteeinheit ein Greenough-Mikroskop verwendet, so ist auch hier ein Konstruktionsweg zu einer weiteren Verkleinerung der Vorrichtung gegeben. Selbstverständlich kann die unten beschriebene Anordnung einer Videobetrachtung mit einem Greenough-Mikroskop auch an anderen Spaltlampengeräten mit entsprechender Anpassung eingesetzt werden. Auch hierbei läßt sich eine Verkleinerung der Vorrichtung erreichen, welche jedoch nicht in dem Masse ausfällt wie unter Verwendung der einsäuligen Halteeinheit.

Es wird in den einen der beiden Strahlengänge des Greenough-Mikroskops zu Darstellung- und Auswertungszwecken ein Teilstrahl ausgeblendet, der auf ein Aufnahmeelement einer Aufnahmeeinheit geführt wird. Würde die Auskopplung des Teilstrahls, wie bisher üblich, im parallelen Strahlengang bei einem einem Greenough-Mikroskop nicht ähnlichen Mikroskop erfolgen, würden sich bedeutend grössere Abmessungen ergeben.

Die gewählte und unten beschriebene Konstruktion gestattet ferner auf einfache Art und Weise optische Filter zu integrieren, welche bessere Beobachtungsergebnisse ermöglichen.

Bei einer bevorzugten Ausführungsvariante lässt sich eine Linsentrageinheit als Zusatz einfach aufstecken. Mit diesem Zusatzteil können insbesondere Untersuchungen am Glaskörper und am Augenhintergrund vorgenommen werden. Diese Untersuchungen wurden bisher mit einem sog. "beweglichen Vorsatzglas nach Hruby" vorgenommen. Diese Einrichtung hatte ein Gestänge, an dem eine Untersuchungslinse verschwenkbar angeordnet war. Das Gestänge hatte eine vertikal verlaufende Führungsstange, welche in Patientenrichtung in einem Schlitz am Spaltlampengerät geführt war. Diese Führungsstange führte durch eine Befestigungsplatte, welche am Kinnhalter für den Patientenkopf befestigt war. Unmittelbar unterhalb der Linse war ein Hebelchen als Handgriff zum Verstellen der Linse angeordnet. Die mit dem bekannten "Vorsatzglas" durchgeführten Untersuchungen waren oftmals nicht reproduzierbar, da beim Loslassen des Hebelchen sich in der Regel die Linse verstellte. Fotografien zur Dokumentation waren somit fast nicht durchführbar.

Weitere Vorteile der Erfindung sowie deren Ausführungsvarianten ergeben sich aus dem untenstehenden Beschreibungstext.

### Kurze Beschreibung der Zeichnungen

Im folgenden werden Beispiele der erfindungsgemässen Vorrichtung bzw. des erfindungsgemässen Spaltlampenmikroskops annand der Zeichnungen näher erfäutert. Es zeigen:
- **Fig. 1**: eine Seitenansicht des erfindungsgemässen Spaltlampengeräts mit Zuordnung zum menschlichen Auge, wobei hier die beispielsweise in **Figur 6** dargestellte Video-Aufnahmeeinheit **46** nicht eingesetzt ist und deshalb die Gehäuseöffnung mit einem Stopfen **50b** verschlossen ist,
- Fig. 2: eine um 90° um eine vertikale Achse gedrehte Ansicht des in **Figur 1** dargestellten Spaltlampengeräts in der dortigen Blickrichtung II,
- Fig. 3: eine Draufsicht ausgehend vom Patientenauge auf eine Halteeinheit des in den **Figuren 1** und **2** dargestellten Spaltlampengeräts,
- Fig. 4: eine schematische Darstellung bekannter Greenough-Mikroskope,
- Fig. 5: einen Querschnitt durch das in der erfindungsgemässen Vorrichtung der **Figuren 1** und **2** eingesetzte abgewandelte Greenough-Mikroskop als Betrachtungseinheit, wobei hier in einer einzigen Abbildung zwei unterschiedliche Lagen optischer Komponenten für je eine unterschiedliche Vergrösserung dargestellt sind,
- Fig. 6: einen Querschnitt entlang der Schnittlinie VI durch einen Strahlengang des in **Figur 5** dargestellten Greenough-Mikroskops zur Darstellung einer Anordnung einer Video-Aufnahmeeinheit, auf deren Aufnahmeelement die eine Teilstrahlung dieses Strahlengangs geführt ist.
- Fig. 7a bis 7f: zwei Ausführungsbeispiele von Anordnungen optischer Komponenten und deren Abstände in den beiden Strahlengängen des in den **Figuren 5** und **6** dargestellten Greenough-Mikroskops, wobei die Bezugszeichen denjenigen in den Figuren entsprechen, die Zahlenangaben sind in Millimetern, O' ist die Objektebene ohne Schutzgias **31, B** die Bildebene für visuelle Betrachtungen und Bv die Bildebene des Videoaufnahmeelements **44;** die **Figuren 7a, 7c** und **7e** zeigen die Lage optischer Komponenten für eine Vergrösserung und die **Figuren 7b, 7d** und **7f** für die andere,
- Fig.8: eine Variante zu der in **Figur 5** dargestellten Betrachtungseinheit,
- Fia. 9: einen Querschnitt entlang der Schnittlinie IX in **Figur 8** zur Darstellung des Strahiverlaufs des aus einem der Beobachtungsstrahlen ausgekoppelten, auf ein Videoaufnahmeelement einer Videoaufnahmeeinneit geführten Teilstrahls,
- Fig. 10: einen Querschnitt durch die in **Figur 9** dargestellte Videoaufnahmeeinheit als separates Bauteil,
- Fig. 11: einen Querschnitt durch eine Beleuchtungseinheit des in **Figur 1** dargestellten Spaltlampenmikroskops,
- Fig. 12: eine Draufsicht auf die in **Figur 11** dargestellte Beleuchtungseinheit,
- Fig. 13: eine Seitenansicht auf die Beleuchtungseinheit mit der in den **Figuren 2** und **12** gezeigten Blickrichtung XIII,
- Fig. 14: eine Draufsicht auf einen in den **Figuren 1** und **2** dargestellten Lenkhebel des Spaltlampengeräts in der in **Figur 1** gezeigten Blickrichtung, wobei eine Abdeckung auf dem Oberteil des Lenkhebels abgenommen ist,
- Fig. 15: das insbesondere in **Figur 1** dargestellte Spaltlampengerät mit einer abnehmbaren Linsentrageinheit.
- Fig. 16: die in **Figur 15** dargestellte Trageinheit als separates Zusatzteil in einer grösseren Darstellung,
- Fig, 17.: die in **Figur 16** dargestellte Trageinheit in der dortigen Blickrichtung XVII und
- Fig. 18: die in **Figur 17** dargestellte Trageinheit in der dortigen Blickrichtung XVIII.

### Wege zur Ausführung der Erfindung

Das in den **Figuren 1** und **2** dargestellte sog. Spaltlampengerät als Vorrichtung zur stereoskopischen Betrachtung eines Auges **1** hat eine Betrachtungseinheit **3** und eine Beleuchtungseinheit **5.** Die Betrachtungseinheit **3** ist mit einer Halteeinheit **23** und die Beleuchtungsainheit **5** mit einer Halteeinheit **7** gehalten. Wie unten ausgeführt wird, ist mit der Beieuchtungsainheit **5** ein Lichtstrahl **9** ais Strahlung erzeugbar, der über einen an der Halteeinheit **7** angeordneten Umlenkspiegel **10** in bzw. auf das Auge **1** führbar ist. Der Querschnitt des Lichtstrahls **9** ist gemäss untenstehenden Ausführungen einstellbar, insbesondere als Lichtstreifen einstellbar. Die Halteeinheit **7** ist über ein Schwenkgelenk mit einer vertikalen Schwenkachse verschwenkbar an einem Vorrichtungsfuss **11** angeordnet.

Die Halteeinheit **7** ist als L-förmiges Bauteil ausgebildet, welches am Ende eines horizontal verlaufenden Schenkels **13** in einem um eine vertikale Achse **14** verschwenkbaren Schwenkgelenk **15** verschwenkbar am Vorrichtungsfuss **11** angeordnet ist. Die Lage der Achse **14** ist derart gewählt, daß sie bei einer an ein (zeichnerisch nur angedeutetes) Stirnband **17** eines (nicht dargestellten) Kopfhalters angelegten menschlichen Stirn an der Augenvorderseite vorbei läuft. Der andere Schenkel **20** des L-förmigen Halteteils **7** verläuft vertikal und ist, wie bereits oben ausgeführt, einsäulig ausgebildet. Damit zwischen der Betrachtungseinheit **3** und dem Patientenauge **1** nur eine geringfügige optische Störung vorhanden ist, ist eine Einsäuligkeit gewählt, welche insbesondere in **Figur 3** dargestellt ist. Im Bereich der Patientenaugenhöhe ist der horizontale Querschnitt der Halteeinheit 5 stark verkleinert. Auf diesem querschnittsverminderten Bereich **18** ist der Umlenkspiegel **10** angeordnet. Der Querschnitt ist so schmal als möglich ausgebildet. Die Verkleinerung des horizontalen Querschnitts wird durch mechanische Stabilitätsvorgaben beschränkt, sowie die für die Strahlführung der Beleuchtung notwendige Breite des Umlenkspiegels **10**. Gegen eine beliebige Breitenverkleinerung des Bereiches **18** spricht femer eine Führung von den unten beschriebenen Elementen innerhalb des Schenkels **20**, der innen hohl ausgebildet ist.

In seinem Hohlraum verläuft ein (nicht dargestellter) stangenförmiger Einstellmechanismus zur Einstellung einer Schlitzbreite in der Beleuchtungseinheit **5,** welche am oberen Ende des Schenkels **(20)** angeordnet ist. Der (nicht dargestellte) stangenförmige Einstellmechanismus.wirkt mit einem nicht dargestellten Exzenter, der innerhalb der Halteeinheit **7** in deren äusserem Bereich der Vereinigung zwischen den beiden Schenkeln **13** und **20** liegt. An jedem der beiden Enden des Exzenters ist ein Einstellknopf**21a** und **21b** angeordnet. Jeder Einstellknopf **21a** und **21b** ist auf seiner Oberfläche griffig ausgebildet.

Die Betrachtungseinheit **3** ist analog zur Beleuchtungseinheit **5** ebenfalls auf einer L-förmigen Halteeinheit **23** angeordnet. Auch diese Halteeinheit **23** hat einen horizontal und einen vertikal liegenden Schenkel **24** und **25**. Das Ende des horizontalen Schenkels **24** ist analog zur Halteeinheit **7** um die vertikale Achse **14** verschwenkbar und mit dem nach unten verlängerten Schwenkgelenk **15** am Vorrichtungsfuss **11** unabhängig gegenüber der Halteeinheit **7** verschwenkbar gelagert. An der Aussenseite des Schenkels **25** ist ein Atemschutzschild **27** auswechselbar gehalten. Am oberen Ende des Schenkels **25** ist die Betrachtungseinheit **3** in einer Höhe angeordnet, welche einen Blick in das Auge **1** erlaubt.

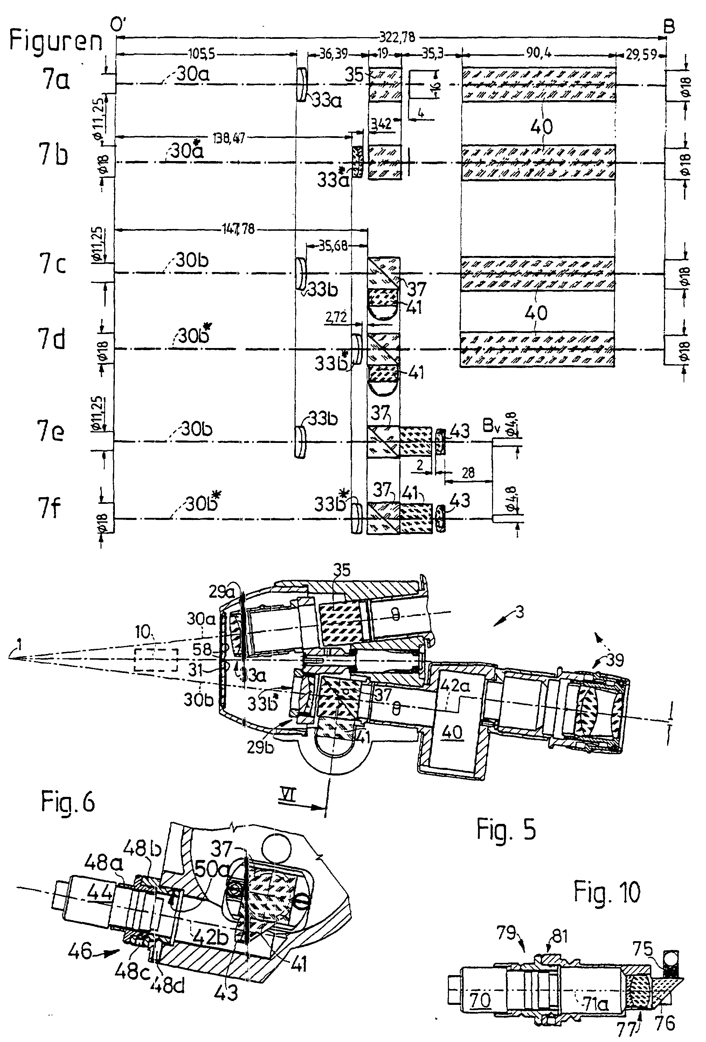

Die Betrachtungseinheit **3** ist prinzipiell als Greenough-Mikroskop ausgebildet. Den prinzipiellen Aufbau eines derartigen Stereomikroskops zeigt **Figur 4** als beispielsweise Abbildung aus Karl Mütze, "ABC der Optik", Suchwort "Stereomikroskopie", 1961, Verlag Werner Dausin, Hanau/Main. Gemäss dieser Literaturstelle dient ein Greenough-Mikroskop zum direkten räumlichen Sehen. Es hat zwei getrennte, um einen Winkel von 14° bis 16° gegeneinander geneigte Mikroskope, wobei dieser Winkel etwa dem Konvergenzwinkel der menschlichen Augenachsen beim Betrachten eines Gegenstands aus der Entfernung der konventionellen Sehweite von 25 cm entspricht. Ein Satz Porro-Prismen **P** erster oder zweiter Art richtet das Bild auf, so dass es in gleicher Lage wie das Objekt gesehen wird. Dies ist nötig, um ein orthoskopisches (tiefenrichtiges) Bild zu erhalten.

Bei einem Greenough-Mikroskop liegen die Objektive sehr dicht beieinander, wodurch gemäss Ausführungen in der obengenannten Literaturstelle keine hohen Aperturen möglich sind. Bei der erfindungsgemässen Vorrichtung wird nun vom typischen Greenough-Mikroskop abgewichen, wie ein Querschnitt in **Figur 5** zeigt. **In Figur 5** werden die beiden voneinander getrennten Einzelmikroskope **29a** und **29b** in einem Querschnitt zueinander geneigt unter einem Winkel von 13° gezeigt. Die Strahlengänge der Einzelmikroskope **29a** und **29b** sind mit **30a** und **30b** gekennzeichnet. Im oberen Figurenteil ist die Lage der optischen Komponenten für einen Vergrösserungsmassstab und in der unteren Bildhälfte für einen anderen gezeigt. Eine Umschaltung der Vergrösserungsmaßstäbe erfolgt mit dem in **Figur 2** zu sehenden Umschalthebel **59.**

Am Beobachtungsstrahleneintritt in das Greenough-Mikroskop **3** ist ein einziges Schutzglas **31** für beide Strahlengänge **30a** und **30b** vor den beiden Objektiven **33a** für den einen Vergrösserungsmaßstab bzw. vor den beiden Objektiven **33b** für den anderen Vergrösserungsmassstab angeordnet. Auf das Objektiv **33a** im "oberen" Strahlengang **30a** folgt eine planparallele Platte **35** zur optischen Anpassung an ein dem Objektiv 33a im "unteren" Strahlengang **30b** nachgeschaltetes Teilerprisma **37.** "Oberer" und "unterer" Strahlengang sind gemäß **Figur 2** linker und rechter Strahlengang. Auf die planparallele Platte **35** folgt ein einem Okular **39,** insbesondere ein einem Wechselokular vorgeschaltetes Porrosches Prisma **36.** Beide Komponenten sind nur im "unteren" Strahlengang dargestellt.

Im Strahlengang **30b** wird eine Bildauskopplung für eine Video-Aufnahmeeinheit **46** vorgenommen. Diese erfolgt mit einem Teilerprisma **37,** welches den Strahlengang **30b** in einen Teilstrahl **42a** über das Porrosche Prisma **40** zum Okular **39** sowie in einen weiteren Teilstrahl **42b** über ein Umlenkprisma **41** und ein Video-Objektiv **43** zu einem Aufnahmeelement **44** der Videoaufnahmeeinheit **46** aufteilt. Die Videoaufnahmeeinheit **46** besteht aus dem Teilerprisma **37**, dem Umlenkprisma **41,** dem Videoobjektiv **43** und dem Videoaufnahmeelement **44.** Das Videoaufnahmeelement **44** ist in einer Fassung **48a** gehalten, welche in einer Justierhülse **48b** steckt. Die Fassung **48a** ist mit einer Klemmschraube **48c** in der Justierhülse **48b** gehalten. Die Justierhülse **48b** sitzt verdreh- und verschiebbar in einer Gehäusebohrung **50a** und ist mit Klemmschrauben **48d** fixierbar, welche in einer umlaufenden Nut am Aussenmantel der Justierhülse **48b** eingreifen. Durch eine Verstellung der Fassung **48a** und der Justierhülse **48b** ist eine optische Bildeinstellung gegeben. Damit kein Staub durch die Gehäusebohrung **50a** eindringen kann, ist diese durch einen abnehmbaren Stopfen **50b** verschlossen. Die Video-Aufnahmeeinheit **46** ist als Ganzes auswechselbar. Ebenso ist das Videoaufnahmeelement auswechselbar.

Neben einer visuellen Betrachtung können somit zusätzlich Videoaufnahmen zur direkten Betrachtung oder zur Aufzeichnung (Dokumentation) vorgenommen werden. Die Anordnung der Aufnahmeeinheit **44** zeigt **Figur 6.**

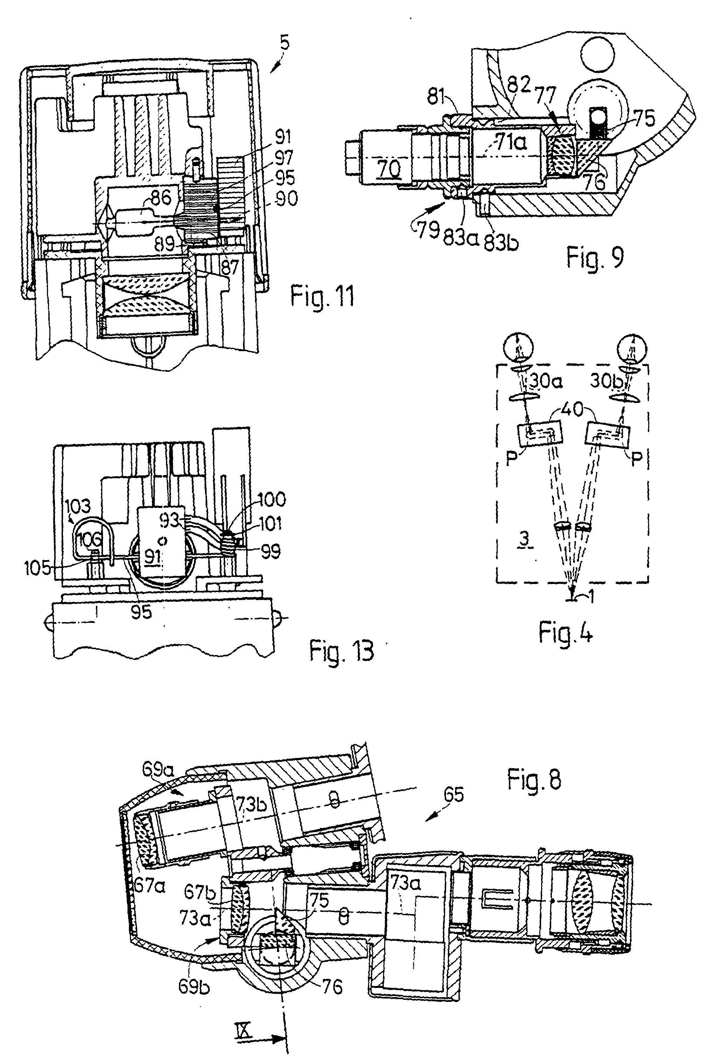

Die **Figuren 7a** bis **7f** zeigen zwei optische Ausführungsvarianten für unterschiedliche Vergrösserungsmassstäbe. Die Figuren **7a, 7c** und **7e** zeigen eine Anordnungsvariante mit je einem Objektiv **33a** bzw. **33b** mit 1,6-facher Vergrösserung und im Video-Strahlengang mit einem Objektiv **43** mit ebenfalls 1,6-facher Vergrösserung.

Bei der anderen Ausführungsvariante, dargestellt in den **Figuren 7b, 7d** und **7f,** sind Komponenten, welche sich von denjenigen in den **Figuren 7a, 7c** und **7e** unterscheiden, mit einem hochgestellten * gekennzeichnet. Bei dieser Ausführungsvariante wird je ein Objektiv **33a'** bzw. **33b'** mit einer 1 : 1-Abbildung sowie im Video-Strahlengang mit einem Objektiv **43'** mit derselben Vergrösserung verwendet. Weitere Ausführungsvarianten sind selbstverständlich möglich.

Die Beleuchtungseinheit **5** hat zwei übereinander angeordnete, um eine horizontale Achse schwenkbare Hebel **45a** und **45b.** Mit diesen Hebeln **45a** und **45b** ist die Höhe und die Breite einer Blendenöffnung einstellbar. Der Querschnitt dieser Blendenöffnung definiert den Querschnitt des auf das Auge **1** zu richtenden Lichtstreifens **9**. Mit diesen beiden Hebeln ist zusätzlich ein Blau- bzw. Graufilter in den Beleuchtungsstrahlengang **9** ein- und ausschwenkbar. Das Ein- bzw. Ausschwenken erfolgt im Endbereich des Schwenkvorgangs des betreffenden Hebels **45a** bzw. **45b.**

Ebenfalls kann ein Gelbfilter **58** mit einer Verstelleinrichtung **47** an der Betrachtungseinheit **3** in die Strahlengänge **30a, 30a*, 30b** und **30b*** eingebracht werden. Das Gelbfilter **58** besteht hier aus zwei Teilbedampfungen der Innenseite des Schutzglases **31.** Mit der Verstelleinrichtung **47** ist das Schutzglas **31** verdrehbar, so dass die beiden Teilbedampfungen **58** einmal vor den Objektiven **33a** und **33b** (in den Strahlengängen **30a** und **30b,** wie in den **Figuren 3** und **5** angedeutet ist) liegen und einmal neben diesen (nicht im Strahlengang **30a** und **30b).**

Wird auf die Augenoberfläche z.B. bei eingesetzter (nicht dargestellter) Kontaktlinse Fluorescein aufgebracht und mit blauem Licht (eingeklapptes Blaufilter) beleuchtet, so tritt gelbe Fluoreszenz auf, welche sich bei einem im Beobachtungsstrahlengang befindlichen Gelbfilter gut mit dem Greenough-Mikroskop **3** beobachten läßt (Passkontrolle von Kontaktlinsen).

An der Betrachtungseinheit **3** ist ein Umschalthebel **59** angeordnet. Mit diesem Umschalthebel 59 sind in den Strahlengängen wechselweise je nach gewünschter Vergrösserung einmal die Objektive **33a** und **33b** bzw. **33a*** und **33b*,** wie sie in den **Figuren 7a, 7c** und **7c** gezeigt sind einschwenkbar und dann in der anderen Hebelstellung dasjenige der Figuren **7b, 7d** und **7f.** Die **Figuren 7e** und **7f** zeigen den Strahlverlauf in einer gegenüber den **Figuren 7a** bis **7d** um 90° geschwenkten Lage. Am Vorrichtungsfuss **11** ist ferner ein Stromanschluss **61** für die Lichtquelle in der Beleuchtungseinheit **5** sowie für die Aufnahmeeinheit **44** vorhanden.

Zur Beobachtung des gesamten Gesichtsfeldes ist unterhalb des unter 45° angeordneten Umlenkspiegels **10** ein nicht dargestellter Kaltlichtleiter angeordnet. In einen Adapter **63** auf dem Gehäuse des Greenough-Mikroskops kann ferner ein Tonometer zum Messen des Augendrucks aufgesetzt werden. Die Helligkeit der "Spaltlampe" in der Beleuchtungseinheit **5** wird durch einen auf den Vorrichtungsfuss **11** angeordneten Handregler **49** eingestellt. Das elektrische Kabel zur Helligkeitsregulierung bzw. Stromversorgung verläuft im Innern der hohlen Halteeinheit **7**. Die Positionierung der Vorrichtung horizontal in X- und Y-Richtung wird mit Hilfe eines am Vorrichtungsfuss **11** angeordneten Lenkhebels **51,** oftmals auch als "Joy-Stick" bezeichnet, vorgenommen. Durch eine seitliche Auslenkung **53** ist der Vorrichtungsfuss **11** seitlich auf einer Achse **52** inY-Richtung **54** verschiebbar. Durch ein nach vom bzw. nach hinten Verschwenken **55** des Lenkhebels **51** ist ein Verschieben in X-Richtung **56** möglich. Die Verschiebung in X-Richtung **56** erfolgt über eine Drehbewegung der an der Achse **52** beidseits angeordneten Räder **57a** und **57b**, welche auf nicht dargestellten Zahnschienen abrollen, die auf einer nicht dargestellten Unterlage befestigt sind. Auf dieser Unterlage ist auch ein das Stirnband **17** aufweisender nicht dargestellter Kopfhalter angeordnet.

Der Lenkhebel **51** ist femer um seine vertikale Achse verdrehbar. Um eine gute Verdrehbarkeit zu erreichen, ist der Lenkhebel **51** in seinem oberen Mantelbereich mit einer umlaufenden Riffelung versehen. Die Verdrehung bewirkt eine synchrone Höhenverstellung der Halteeinheiten **7**und **23** und damit eine Höhenverstellung des in das Auge **1** zu richtenden Lichtstreifens **9** zusammen mit der Betrachtungseinheit **5**.

Da der Schenkel **20** der Halteeinheit **7** sehr schlank auszubilden ist, kann aus Herstellungsgründen davon abgesehen werden, das Stromkabel in ihm zu verlegen. Die Stromversorgung geht in diesem Fall zum Stromanschluss **61,** von diesem zum Handregler **49** und von diesem dann wieder zurück zum Stromanschluss **61** und von diesem dann über ein externes (nicht dargestelltes Kabel) über die (ebenfalls nicht dargestellte Kopfstütze) in die Beleuchtungseinheit **5**.

Anstatt die Auskopplung für die Aufnahmeeinheit **44**, wie oben beschrieben, mit teildurchlässigen Komponenten beispielsweise über das Teilerprisma **37** auszuführen, kann auch nur ein Bruchteil des Strahlquerschnitts unter Verwendung eines Auskoppelspiegels oder eines Auskoppelprismas ausgekoppelt werden, wie beispielsweise in den **Figuren 8** bis **10** dargestellt ist. **Figur 8** zeigt eine Variante **65** zu der in **Figur 5** dargestellten Betrachtungseinheit **3** (Greenough-Mikroskop). Die Eingangsobjektive **67a** und **67b** der Einzelmikroskope **69a** und **69b** und deren Lage ist analog zu den Objektiven **33a** und **33b*** in **Figur 5** ausgebildet. Da eine Auskopplung eines Teilstrahls **71a** zu einem zum Videoaufnahmeelement **44** analog ausgebildeten Videoaufnahmeelement**70** (sichtbar in den **Figuren 9** und **10)** durch Auskopplung eines Bruchteils des einfallenden Strahls **73a** (analog zum Strahl **30b**) erfolgt, ist eine optische Kompensation durch eine planparallele Platte analog zur Platte **35** nicht notwendig. Hierdurch wird der Aufbau der Betrachtungseinheit **65** gegenüber der Betrachtungseinheit **3** wesentlich vereinfacht.

Zur Auskopplung eines Teilstrahls **71a** wird ein Prisma **75** verwendet, welches in den Querschnitt des Strahls **73a** teilweise hineinragt. Der ausgekoppelte Teilstrahl **71a** wird mit einem zweiten Prisma **76** ein weiteres Mal umgelenkt und mit einer Abbildungsoptik (Videoobjektiv) **77** auf der Empfangsebene des Videoaufnahmeelements **70** abgebildet. Die Videoaufnahmeeinheit **79** besteht hier aus dem einen Teilstrahl geometrisch auskoppelnden Prisma **75,** dem Prisma **76,** der Abbildungsoptik **77** und dem Videoaufnahmeelement **70.**

Die in den **Figuren 9** und **10** dargestellte Videoaufnahmeeinheit **79** (Kamera) ist ebenfalls als Ganzes auswechselbar; es kann aber auch nur das Videoaufnahmeelement 70 alleine ausgewechselt werden. Die Prismen 75 und 76, die Abbildungsoptik77 und das Videoaufnahmeelement **70** sind in einem Gehäuse **81** angeordnet und gehalten, welches optisch passend in eine Gehäuseöffnung **82** der Betrachtungseinheit **65** derart einschiebbar ist, dass das Prisma **75** richtig in den Strahl **73a** zur Auskopplung des Teilstrahls **71a** zu liegen kommt. Auch hier ist ein Verschieben und Verdrehen des Vidoaufnahmeelements **70** zur Bildeinstellung gegeben. Das Gehäuse **81 (Figur 9)** hat analog zur Darstellung in **Figur 6** ebenfalls eine Fassung für das Videoaufnahmeelement **70** und eine Justierhülse. Die Fixierung erfolgt auch hier mit Klemmschrauben**83a** und **83b.**

Durch die Auswechselbarkeit der Videoaufnahmeeinheit **79** ist u.a. eine verkaufstechnisch einfache Nachrüstung der Betrachtungseinheit **65** mit dieser Videoaufnahmeeinheit **79** gegeben. Ferner ist nach dem Herausnehmen der Videoaufnehmeeinheit **79** der Bildkontrast in beiden Beobachtungsstrahlengängen **73a** und **73b** gleich. Die Betrachtungseinheit **65** ist gegenüber der Betrachtungseinheit **3** einfacher und damit auch kostengünstiger herzustellen.

Die Anordnung einer in die Beleuchtungseinheit **5** eingesetzten Lichtquelle **86** zeigt **Figur 11** in vergrössertem Querschnitt. Als Lichtquelle **86** wird eine sog. Hochtemperaturquarzlampe verwendet, welche in einem Passsockel **87** auswechselbar gehalten ist. Der Sockel **87** sitzt mit einer Spielpassung in einer Buchse **89.** Der Sockel **87** hat Kontaktstifte **90,** welche in passende Buchsen eines vom Sockel **87** abziehbaren Stekkerstückes **91** greifen. Vom Steckerstück **91** geht ein Kabel **93** zu einem elektrischen Anschlussstück **94.** Gegen Herausrutschen ist der Sockel **87** mit einem federnden Bügel **95** aus Federdraht gehalten, der in einer Rille **97** des Sockels **87** liegt. Der Bügel **95** ist an seiner einen Seite annähernd kreiszylindrisch, mit hier beispielsweise fünf Windungen, ein "Rohrstück" **99** bildend, gewickelt. Das "Rohrstück" **99** steckt auf einem Stift **100,** dessen oberes Ende eine Klemmscheibe (Seegerring) **101** trägt, der ein Herausrutschen des "Rohrstücks" **99** und damit des Bügels **95** verhindert.

Das andere Ende des Bügels **95** hat eine Griffschlaufe **103,** welche in eine umlaufende Rille **105** im Oberteil eines Stiftes **106** einlegbar ist. Der Bügel **95** ist nun derart federnd vorgebogen, dass er den Sockel **87** in die Buchse **89** und sich selbst gegen die Rille **105** drückt. Zum Auswechseln der Lichtquelle **86** muss das Steckerstück **91** abgezogen werden und anschliessend die Griffschlaufe **103** nur über das obere Ende des Stiftes **106** gehoben werden. Die Lichtquelle **86** kann nun mit dem Sockel **87** herausgezogen werden. Damit der Sockel **87** gut greifbar ist, steht er etwas über den äusseren Rand der Buchse **89** hervor.

Vorteil der Anordnung zur Lichtquellenhalterung ist deren einfache Ausgestaltung. Ferner wird zum Lichtquellenwechseln kein Werkzeug benötigt.

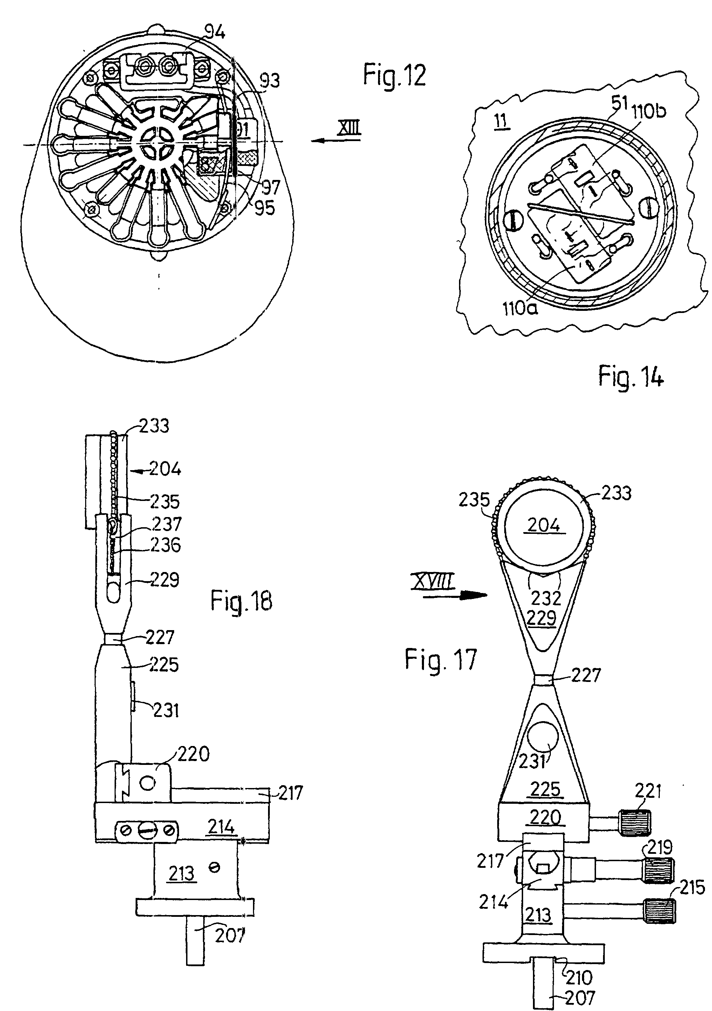

Im Oberteil **109** des Lenkhebels **51** sind, wie in **Figur 14** angedeutet, Schaltelemente **110b** und **110b** zum Steuern von Funktionen der Vorrichtung oder von Funktionen, die periphere Einheiten steuern, welche mit dem Betrachtungsvorgang zusammenhängen. In der hier dargestellten Ausführungsform sind im Oberteil **109** als Schaltelemente zwei nebeneinander liegende Mikroschalter (micro-switch, Kippschalter, ...) **110a** und **110b** als signalgebende Elemente angeordnet. Die beiden Mikroschalter **110a** und **110b** sind von der Oberseite **111** des Oberteils **109** her bevorzugt mit dem Daumen bedienbar. Soll die Vorrichtung in einer rauhen Umgebung eingesetzt werden, so ist die Oberseite **111** mit einer elastischen Folie spritzwasserdicht abgedeckt.

Anstelle von Mikroschaltern können auch Taster bzw. Kipptaster verwendet werden. Drückt man beispielsweise den oberen Teil des als Kipptaster ausgebildeten Schalters **110a,** so kann beispielsweise über einen nicht dargestellten Motorantrieb die Lichtspaltbreite der Lichtquelle verringert werden. Drückt man dann den unteren Teil des Schalters **110a,** würde der Spalt vergrössert werden. Diese Funktion würde eine Handbedienung der Einstellknöpfe **21a/b** durch eine weitere Hand eliminieren. Über den Schalter **110b** könnte man dann in analoger Weise die Helligkeit steuern, was eine Elimination der Einstellung über den Handregler **49** ergeben würde. Der behandelnde Arzt kann dann laufend beobachten, ohne einen Suchblick auf diese Einstellelemente nehmen zu müssen. Auch hat der Arzt die früher zur Einstellung benötigte Hand für Behandlungsmanipulationen frei.

Mit diesen beiden Schaltern/Kipptasten **110a/b** können andere Einheiten eingestellt werden. Eine elektrische sowie signaltechnische Verbindung könnte über den Anschluss **61** oder über einen separaten nicht dargestellten Anschluss erfoigen. Es könnte z. B. ein Tonometer an die Augenoberfläche herangefahren werden.

Durch Betätigen der beiden Schalter/Kipptasten **110a/b** können mit Motorantrieben Verstellungen vorgenommen werden. Damit nun der Arzt weiss, in welcher Stellung sich gerade die betreffende Einheit, bzw. die Spaitbreite oder die Helligkeit befinden, kann eine Einspiegelung von Daten in den Strahlengang der Betrachtungseinheit **3** bzw. **65** vorgenommen werden. Die Einspiegelung würde nun analog zu einer Strahlausspiegelung für das Videoaufnahmeeiement **44** bzw. **70** erfolgen. Anstelle des Videoaufnahmeelements **44** bzw. **70** hätte man nun ein Anzeigeelement, dessen Biidinformation eingespiegelt wird. Zur Einspiegelung ist dann gegenüber den Darstellungen in den **Figuren 5** und **8** das Prisma **37** bzw. **75** um 180° zu drehen.

Soll die erfindungsgemässe Vorrichtung (Spaltlampengerät) auch noch zur bevorzugten Untersuchung des Glaskörpers und des Augenhintergrunds des Patienten verwendet werden, so wird die Vorrichtung mit einer ohne Verwendung von Werkzeugen manuell über eine Kupplung **201** anbring- und wieder abnehmbare Linsentrageinheit **203** mit einer Untersuchungslinse **204** vor den Beobachtungsstrahleneingang in die Betrachtungseinheit **3** in den Beobachtungsstrahlengang, also vor das Schutzglas **30,** eingebracht. Die Untersuchungslinse **204** ist selbsthaltend verdreh- sowie selbsthaltend in allen Raumrichtungen verstellbar mit der Linsentrageinheit **203** gehalten. Die Linsentrageinheit **203** hat im Gegensatz zu dem bekannten, mit einer Spaltlampe zusammen einsetzbaren Vorsatzglas von Hruby keine mechanische Verbindung mit dem Kopf- bzw. Kinnhalter des Patienten.

Die Linsentrageinheit **203** hat einen tellerförmigen Auflageteil **205,** aus dem ein zylindrischer Bolzen **207** hervorsteht. Der Querschnitt des Bolzens **207** ist derart gewählt, dass er mit einer Spielpassung in eine in **Figur 1** dargestellte Achsbohrung **209** einschiebbar ist. Die Achsbohrung **209** ist zentrisch zur vertikalen Achse des Schwenkgeienks **15** ausgebildet. Mit den Schwenkgelenk **15** sind der Halteteil **7** für die Beleuchtungseinheit 5 und der Halter **23** für die Betrachtungseinheit **3** verschwenkbar. Der Bolzen **207** und die Achsbohrung **209** bilden eine Steckkupplung **201.**Die Verdrehsicherheit der Linsentrageinheit **203** wird dadurch erreicht, dass der Tellerrand des Auflageteils **205** mit einer Auskerbung **210** versehen ist. In diese Auskerbung **210** greift im eingesteckten Zustand der hervorstehende Teil eines an der Vorderseite des horizontalen Schenkels **13** der Halteeinheit **7** angeordneten Blechstreifens **211.** Der Auflageteil **205** hat in Verlängerung nach oben einen annähernd quaderförmigen Basisteil **213,** auf dessen horizontal liegenden Oberseite ein erster Schlitten **214** verschiebbar in Quaderlängsrichtung (im montierten Zustand in Richtung auf das Patientenauge 1 hin bzw. von diesem weg) angeordnet ist. Die Führung des Schlittens **214** auf dem Basisteil **213** erfolgt beispielsweise in einer Schwalbenschwanzführung, die mit einer zur besseren Griffigkeit mit einer Rändelung versehenen Klemmschraube **215** fixierbar ist. Bei gelöster Klemmschraube **215** ist eine Verschiebung per Hand möglich. Mit dieser Führung ist eine Grobeinstellung des Abstandes der Linse **204** vom Patientenauge **1** durchführbar. Auf dem ersten Schlitten **214** sitzt ein zweiter Schlitten **217**, der in dieselbe Richtung wie der erste Schlitten **214** verschiebbar ist. Die Verschiebung erfolgt jedoch über eine ebenfalls gerändelte Feineinstellschraube **219.** Horizontal, senkrecht zum ersten bzw. zweiten Schlitten **214** bzw. **217** ist ein dritter Schlitten **220** angeordnet, der ebenfalls über eine Feineinstellschraube **221** durch deren Verdrehen verschiebbar ist. Mit den beiden Feineinstellschrauben **219** und **221** erfolgt eine Feineinstellung der Untersuchungslinse **204** in der horizontalen Ebene.

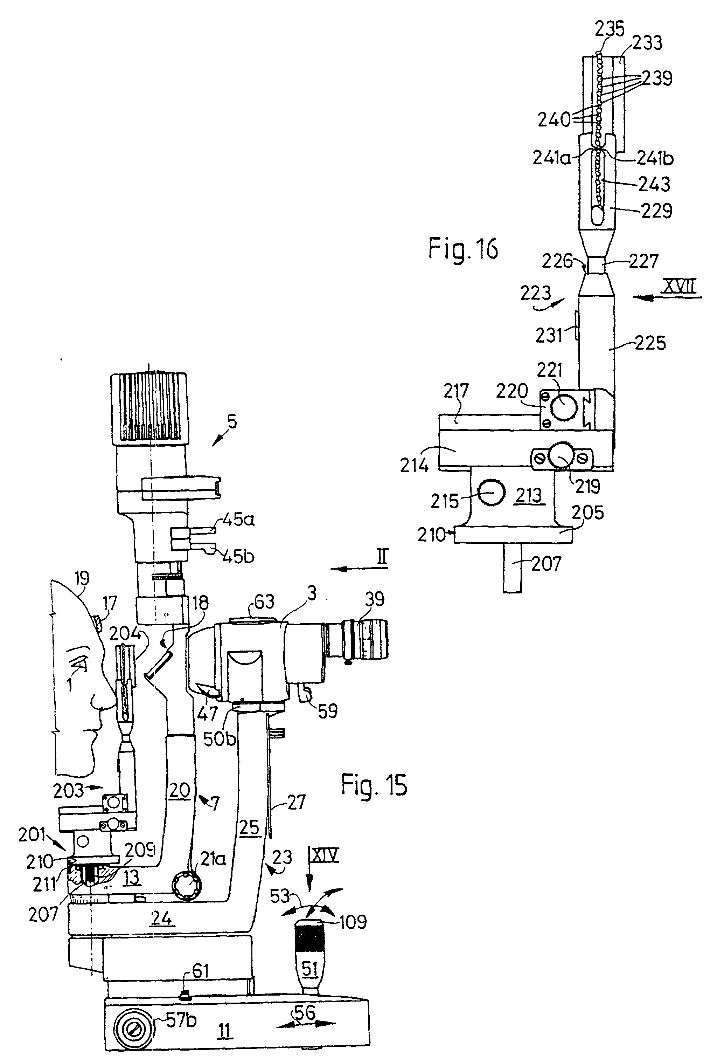

Zur vertikalen Höhenverstellung ist ein zweiteiliger Linsenständer **223** vorgesehen, der an seinem oberen Ende die Untersuchungslinse **204** trägt. Der untere Teil **225** sitzt auf dem dritten Schlitten **220** und verjüngt sich gegen oben prismatisch. Vom oberen Ende des Teils **225** verläuft zentrisch in der Symmetrieachse eine Sackbohrung **226** in den Teil **225** hinein. In dieser Sackbohrung **226** steckt ein Dorn **227**, der in das obere Teilstück **229** des Linsenständers **223** übergeht. Ausgehend vom Domansatz verbreitert sich das Teilstück **229** prismatisch nach oben. Der Dorn **227** ist in der Sackbohrung **226** verschiebbar. Durch diese Verschiebung wird die vertikale Höhe der Untersuchungslinse **204** manuell eingestellt. Diese Höhenverschiebung ist auf Grund eines reibungsbehafteten Kraftschlusses selbsthemmend. Die Selbsthemmung wird durch einen unverlierbar im unteren Teil 225 angeordneten Permanentmagneten erreicht, der sich jedoch in Richtung auf die Oberfläche des Doms **227** hin bewegen kann. Da der Dorn **227** aus ferromagnetischem Material besteht, wird der Permanentmagnet bei in das Sackloch **226** eingestecktem Dorn **227** gegen dessen Oberfläche gezogen und blockiert damit selbsthemmend die Höhenverschiebung. Die Blockierung ist jedoch nur so stark, dass eine Verschiebung auf Grund des Eigengewichts von Untersuchungslinse **204** plus oberem Teilstück **229** unterbunden; eine Verstellung aber dennoch manuell möglich ist. Die Lage des Permanentmagnets ist in den **Figuren 16** bis **18** durch die scheibenartige Montagehilfe **231** ersichtlich.

Die Untersuchungslinse **204** liegt in einer V-förmigen Ausnehmung **232** am oberen Ende des Teilstücks **229** an ihrem Fassungsmantel **233** auf. Gehalten wird die Untersuchungslinse **204** mit einem bandartigen, eine kettenartige Struktur aufweisenden flexiblen Lisenhalteelement **235.** Ein Ende des Elements **235** ist mit einer Feder **236** annähernd mittig am Ende einer Seitenlängsnut **237** des Teilstücks **229** gehalten. Das Element **235** hat als kettenartige Struktur in Längsrichtung gleich distanzierte Noppen **239,** welche durch Zwischenräume **240** mit einem dünneren Bandquerschnitt getrennt sind. Einer dieser Zwischenräume **240** wird zwischen zwei Nasen **241a** und **241** b in einer Seitenlängsnut **243** auf der der Seitenlängsnut **237** gegenüberliegenden Seite eingehakt. Die Feder **236** spannt das Linsenhalteelement **235** und zieht dadurch die Untersuchungslinse **204** fixierend in die Ausnehmung **232.**

Das Linsenhalteelement **235** kann gegenüber den Noppen **239** und den schmalen Zwischenräumen **240** auch eine andere Struktur, wie beispielsweise eine Ausbildung als Kette aufweisen. Bei der Verwendung einer Kette könnten ebenfalls die beiden Nasen **241a** und **241b** vorhanden sein, man würde dann die Kette an ihren Aussenbereichen einhängen; es könnte aber auch nur eine einzige Nase vorhanden sein, in die dann jeweils ein Kettenglied eingehängt würde.

Wie oben ausgeführt, wird die Verdrehsicherheit der Linsentrageinheit **203** mit der Auskerbung **210** und einem hierzu passenden Blechstreifen **211** an der Halteeinheit **7** vorgenommen. Es können aber ebensogut andere Rastelemente vorgesehen werden, wie beispielsweise ein zum Bolzen **207** radial angeordneter Stift, der in eine entsprechende Bohrung in der Halteeinheit **7** greift. Die Orte von Stift und Bohrung können selbstverständlich vertauscht sein. Es können auch strukturierte Oberflächen verwendet werden, deren Strukturen ineinander greifen.

Um störende Reflexe bei der Untersuchung zu beseitigen sowie um den Beobachtungsstrahlengang abzulenken, kann der obere Teil **229** des Linsenständers mit einer Verkippeinrichtung für die Untersuchungslinse **204** ausgerüstet werden. Die Verkippeinrichtung kann eine einfache Schwenkachse sein. Bevorzugt wird man aber drei voneinander distanzierte Schwenkgelenke verwenden, deren Schwenkachsen parallel zueinander verlaufen. D.h. man hat einen Winkelschenkel mit einem einstellbaren Scheitelwinkel, wobei die anderen Schenkelenden wiederum mit einem Schwenkgeienk verschwenkbar sind. Am obersten Schenkelende ist das die Untersuchungslinse **204** verschwenkbar gehalten. Mit dieser Anordnung ist ein Verkippen der Linse unter Beibehaltung des Linsenmittelpunkts im einem vorgegebenen Raumpunkt möglich.

Durch den Einsatz der Linsentrageinheit **203** kann der Arzt insbesondere über deren räumliche Feineinstellung die Untersuchungslinse **204** optimal auf das Patientenauge **1** einstellen. Nach der Einstellung hat er beide Hände für auszuführende Untersuchungen und Behandlungen frei. Auch kann er, insbesondere unter Verwendung der Videoaufnahmeeinheit **46** bzw. **70**, entsprechende Dokumentationen vornehmen. Anstelle der Videoaufnahmeeinheit kann auch ein Fotoapparat angeflanscht werden, um entsprechende Dokumentationen vorzunehmen. Da die Untersuchungslinse einmal eingestellt selbsthaltend in ihrer Lage verbleibt, kann in aller Ruhe die Aufnahme unter Auswahl des Bildausschnittes und den Scharfeinstellungen vorgenommen werden.

Erst die erfindungsgemässe Ausgestaltung des vertikal verlaufenden Astes **20** der Halteeinheit **7** einsäulig mit dem schmalen Säulenquerschnitt gestattet eine optimale Untersuchung des Glaskörpers sowie des Augenhintergrunds unter Verwendung der durch die Linsentrageinheit **203** getragenen Untersuchungslinse **204.** Man kann die Linsentrageinheit **203** auch mit dem eingangs beschriebenen bereits bekannten Spaltlampengerät, welches eine dreisäulige Halteeinheit hat, verwenden. Auch ist ein Einsatz zusammen mit anderen Spaltlampengeräten möglich, sofern eine entsprechende Kupplung vorhanden ist

## Patentansprüche

1. Vorrichtung, insbesondere ein Spaltlampengerät, zur stereoskopischen Betrachtung eines Patientenauges **(1)** mit einer Betrachtungseinheit **(3, 65),** wobei das Auge **(1)** mit einem von einer Beleuchtungseinheit **(5)** ausgehenden Lichtstreifen **(9)** mit vorgegebenem Querschnitt beleuchtbar, die Beleuchtungseinheit **(5)** oben auf einem vertikal verlaufenden Ast **(20)** einer Halteeinheit **(7)** angeordnet, das zu betrachtende Auge **(1)** in einer annähernd horizontal verlaufenden Ebene auf einer Seite der Halteeinheit **(7)** positionierbar und die Betrachtungseinheit **(3)** annähernd in der Ebene auf der hierzu gegenüberliegenden Seite der Halteeinheit **(7)** angeordnet ist, **dadurch gekennzeichnet, dass** der die Beleuchtungseinheit **(5)** tragende, vertikal verlaufende Ast **(20)** der Halteeinheit **(7)** einsäulig mit einem schmalen Säulenquerschnitt ausgebildet ist, auf dem ein Umlenkspiegel **(10)** angeordnet ist, mit dem die Strahlung **(9)** der Beleuchtungseinheit **(5)** ins bzw. auf das Auge **(1)** richtbar ist, wodurch ein guter Patienten-Arzt-Kontakt mit lediglich einer geringfügigen optischen Störung zwischen Betrachlungseinheit **(3)** und dem Patientenauge **(1)** erreichbar ist.

2. Vorrichtung nach Anspruch 1, **dadurch gekennzeichnet, dass** die Halteeinheit **(7)** L-förmig, bevorzugt einteilig ausgebildet ist, wobei die Beleuchtungseinheit (5) am einen Schenkelende angeordnet und der andere Schenkelendbereich in einem eine vertikale Schwenkachse **(14)** aufweisenden Schwenkgelenk **(15)** gehalten ist.

3. Vorrichtung nach Anspruch 1 oder 2, **gekennzeichnet durch** eine zweite L-förmig, bevorzugt einteilig ausgebildete Halteeinheit **(23)** für die Betrachtungseinheit **(3),** wobei die Betrachtungseinheit **(3)** am einen Schenkelende angeordnet und der andere Schenkelendbereich in einem eine vertikale Schwenkachse **(14)** aufweisenden Schwenkgelenk **(15)** gehalten ist und insbesondere die Schwenkachse **(14)** für die Halteeinheiten **(7, 23)** der Betrachtungseinheit **(3)** und der Beleuchtungseinheit **(5)** zusammenfallen.

4. Vorrichtung nach einem der Ansprüche 1 bis 3, **dadurch gekennzeichnet, dass** die Halteeinheit **(7)** für die Beleuchtungseinheit **(5)** innen hohl mit einem Hohlraum ausgebildet, ein Kraftübertragungselement zur Querschnittsverstellung einer Blendenöffnung in der Beleuchtungseinheit **(5)** im Hohlraum zur Erzeugung eines vorgegebenen Lichtstreifenquerschnitts geführt und ein auf das Kraftübertragungselement wirkendes Einstellelement **(21a, 21b)** bevorzugt im Schenkelursprung der ersten L-förmigen Halteeinheit **(7)** angeordnet ist.

5. Vorrichtung, nach einem der Ansprüche 1 bis 4 **dadurch gekennzeichnet, dass** das Stereomikroskop ein keinen kollimierten (nichtparallelen) Strahlengang aufweisendes Greenough-Mikroskop ist, aus dessen wenigstens einem Strahlengung **(30b, 73a)** mit einer Bildein- und/oder-auskoppeleinheit **(37; 75, 76)** ein Teilstrahl **(42b, 71a)** ausblendbar ist, dessen Bildinformation bevorzugt zu einer in der Betrachtungseinheit **(3, 65)** angeordneten Aufnahmeeinheit **(46, 79)** geführt ist.

6. Vorrichtung nach Anspruch 5, **dadurch gekennzeichnet, dass** die Aufnahmeeinheit **(79)** in die Betrachtungseinheit **(65)** ein- bzw. aussteckbar ausgebildet ist, und bevorzugt die Auskopplung des Teilstrahls **(71a)** zur Aufnahmeeinheit **(79)** durch eine geometrische Strahlaufteilung erfolgt.

7. Vorrichtung, nach einem der Ansprüche 1 bis 6, **dadurch gekennzeichnet, dass** zur Positionierung in X und Y-Richtung (d.h. in einer horizontalen Ebene) und bevorzugt in Z-Richtung (Höhenverstellung) des in das Patientenauge **(1)** zu richtenden Lichtstreifens **(9)** zusammen mit der Betrachtungseinheit **(3)** ein Lenkhebel **(51)** vorhanden ist, in dessen Oberseite signalgebende Elemente **(110a, 110b)** eingesetzt sind, durch deren Betätigung Funktionen der Vorrichtung und/oder von mit der Vorrichtung zusammenwirkenden peripheren Einheiten steuerbar sind, damit die betrachtende Person ihren Blick nicht von der Betrachtungseinheit **(3)** abwenden muss, sowie bevorzugt manuelle Einstelltätigkeiten auch mit der den Lenkhebel **(51)** bedienenden Hand gleichzeitig vorgenommen werden können.

8. Vorrichtung nach Anspruch 5 oder 6, insbesondere nach Anspruch 7, **gekennzeichnet durch** ein Zeicheninformationen erzeugendes Element und eine optische Einkoppeleinheit, mit der das Bild in wenigstens einen Strahlengang der Betrachtungseinheit zur Beobachtung mit dem Okular der Betrachtungseinheit einblendbar ist und die Bildinformation bevorzugt Daten anzeigt, welche insbesondere **durch** Betätigen des Lenkhebels eingestellt bzw. verändert werden.

9. Vorrichtung nach einem der Ansprüche 1 bis 8, **dadurch gekennzeichnet, dass** die Beleuchtungseinheit **(5)** wenigstens ein in den Beleuchtungsstrahl einbringbares optisches Filter, insbesondere ein Blau- und/oder ein Graufilter und bevorzugt die Betrachtungseinheit **(3)** wenigstens ein in den Betrachtungsstrahlengang einbringbares weiteres Filter, insbesondere ein Gelbfilter **(58)** hat.

10. Vorrichtung **gekennzeichnet durch** eine ohne Verwendung von Werkzeugen manuell über eine verdrehsichere Steckkupplung **(201)** anbring-und wieder abnehmbare Linsentrageinheit **(203)** mit einer Untersuchungslinse **(204),** weiche dann vor dem Beobachtungsstrahleneingang in die Betrachtungseinheit **(3)** im Beobachtungsstrahlengang zu liegen kommt und insbesondere zur Untersuchung des Glaskörpers und des Augenhintergrunds des Patientenauges **(1)** dient, die Untersuchungslinse **(204)** selbsthaltend verdreh- und selbsthaltend in allen Raumrichtungen verstellbar mit der Linsentrageinheit **(203)** gehalten ist, wobei die Trageinheit **(203)** keine mechanische Verbindung zu der Vorrichtung benachbarten anderen Vorrichtungen, wie beispielsweise einem Kopf- und Kinnhalter, hat.

11. Vorrichtung nach Anspruch 10 und bevorzugt nach Anspruch 2, **dadurch gekennzeichnet, dass** die Kupplung **(201)** als verdrehsichere Steckkupplung **(207, 209, 210, 211)** in vorteilhafterweise mit einem Auflageteil **(205)** für die Auflage auf der Halteeinheit **(7)** ausgebildet ist, wobei ein Kupplungsteil der Kupplung **(201)** in der Halteeinheit **(7)** bevorzugt als zentrische Achsbohrung **(209)** zur vertikalen Schwenkachse **(14)** der Halteeinheit **(7)** ausgebildet ist, wobei in die Achsbohrung **(209)** ein passender Bolzen **(207)** der Linsentrageinheit **(203)** einsteckbar ausgebildet ist, und die Verdrehsicherheit bevorzugt durch wenigstens ein Rastelement **(210, 211)** mit je einem Rastteilelement **(211)** an der Halteeinheit **(7)** und einem weiteren **(210)** an der Linsentrageinheit **(203)** gebildet sind, wobei beide Teilelemente **(210, 211)** ineinander steckbar sind.

12. Vorrichtung nach einem der Ansprüche 10 oder 11, **gekennzeichnet durch** einen Kupplungsteil **(207),** mit dem die Linsentrageinheit **(203)** ohne Verwendung von Werkzeugen manuell auf einer Halteeinheit (7) für eine Beleuchtungseinheit (5) einer Vorrichtung nach einem der Ansprüche 1 bis 12 aufsetzbar ist, eine Untersuchungslinse (204), bevorzugt geeignet zur Untersuchung des Glaskörpers und des Augenhintergrundes eines Patientenauges **(1),** einen zweiteiligen Linsenständer **(223),** dessen einer Teil **(229)** gegen den anderen **(225)** selbsthaltend zur Höhenverstellung der Untersuchungslinse **(204)** verschiebbar ist, sowie eine Verschiebeeinrichtung (214, 217, 220) zur Verschiebung des Linsenständers (223) in zwei zueinander senkrechten horizontalen Richtungen.

13. Vorrichtung nach Anspruch 12, **dadurch gekennzeichnet, dass** der eine Teil **(229)** des Linsenständers **(223)** einen Dom **(227)** aufweist, der mit einer Spielpassung in einem Sackloch **(226)** des anderen Teils **(225)** des Linsenständers **(223)** verschiebbar ist, der Dom **(227)** bevorzugt aus ferromagnetischem Material besteht, in dem Sackloch **(226)** ein begrenzt beweglicher und damit unverlierbarer Permanentmagnet **(231)** angeordnet ist, der gegen die Oberfläche des ins Sackloch **(226)** eingeschobenen Doms **(227)** drückt und somit den einen Teil **(229)** des Linsenständers **(223)** in einer einmal eingestellten Position hält.

14. Vorrichtung nach Anspruch 12 oder 13, **gekennzeichnet durch** ein bandartiges, eine kettenartige Struktur aufweisendes Linsenhalteelement **(235)** dessen eines Ende in Elementlängsrichtung am einen Teil **(229)** des Linsenständers **(223)** mit einem Federelement **(236)** spannbar gehalten ist, eine Linsenauflage **(232)** für die Untersuchungslinse **(204)** ebenfalls an dem einen Teil **(229)** des Linsenständers **(223)** und ein Einhängeelement **(241a, 241b)** für den Endbereich des um den Linsenrand **(233)** spannbaren Linsenhalteelements **(235),** damit die Untersuchungslinse **(204)** auf einfache Art und Weise gegen eine andere Untersuchungslinse bevorzugt mit anderer Brennweite austauschbar ist.

15. Vorrichtung nach einem der Ansprüche 12 bis 14, **gekennzeichnet durch** eine Linsenkippeinheit zur Verkipplung der Untersuchungslinse **(204),** wobei die Verkippeinheit drei voneinander distanzierte Schwenkgelenke mit zueinander parallelen Schwenkachsen aufweist, damit der Linsenmittelpunkt beim Verkippen der Linse an einem vorgegebenen Raumpunkt gehalten werden kann.

## Claims

1. Apparatus, especially a split-lamp device, for the stereoscopic viewing of a patient's eye (1) with a viewing unit (3, 65), the eye (1) being illuminable with a light beam (9) which originates from an illumination unit (5) and which has a predetermined cross-section, the illumination unit (5) being arranged at the top of a vertically extending branch (20) of a holding unit (7), the eye (1) to be viewed being positionable in an almost horizontally extending plane on one side of the holding unit (7), and the viewing unit (3) being arranged approximately in the plane on the opposite side of the holding unit (7), **characterised in that** the vertically extending branch (20) of the holding unit (7), which branch carries the illumination unit (5), is in the form of a single column having a narrow column cross-section on which is arranged a deflecting mirror (10) by means of which the radiation (9) of the illumination unit (5) can be directed into or onto the eye (1), as a result of which good patient-doctor contact can be achieved with only slight optical disturbance between the viewing unit (3) and the patient's eye (1).

2. Apparatus according to claim 1, **characterised in that** the holding unit (7) is L-shaped and preferably in one piece, the illumination unit (5) being arranged at the one branch end and the other branch end region being held in a pivot hinge (15) having an upright pivot axis (14).

3. Apparatus according to claim 1 or 2, **characterised by** a second L-shaped, preferably one-piece, holding unit (23) for the viewing unit (3), the viewing unit (3) being arranged at the one branch end and the other branch end region being held in a pivot hinge (15) having an upright pivot axis (14), and, in particular, the pivot axes (14) for the holding units (7, 23) of the viewing unit (3) and of the illumination unit (5) coinciding.

4. Apparatus according to any one of claims 1 to 3, **characterised in that** the holding unit (7) for the illumination unit (5) is in a form which is hollow on the inside with a cavity, a power transmission member for the cross-sectional adjustment of a diaphragm opening in the illumination unit (5) is guided in the cavity in order to generate a predetermined light beam cross-section, and an adjusting member (21a, 21b) which acts on the power transmission member being arranged preferably in the point of origin of the branch of the first L-shaped holding unit (7).

5. Apparatus according to any one of claims 1 to 4, **characterised in that** the stereoscopic microscope is a Greenough microscope which does not have a collimated (non-parallel) beam path, and a sub-beam (42b, 71a) can be blanked out of the at least one beam path (30b, 73a) with an image-coupling and/or -decoupling unit (37; 75, 76), the sub-beam's (42b, 71a) image information preferably being guided to a recording unit (46, 79) arranged in the viewing unit (3, 65).

6. Apparatus according to claim 5, **characterised in that** the recording unit (79) is in a form which can be plugged into and unplugged from the viewing unit (65), and, preferably, the decoupling of the sub-beam (71a) relative to the recording unit (79) is effected by geometric beam-splitting.

7. Apparatus according to any one of claims 1 to 6, **characterised in that**, for the positioning of the light beam (9) to be directed into the patient's eye (1), together with the viewing unit (3), in the X and Y direction (that is to say, in a horizontal plane) and preferably in the Z direction (height adjustment), there is present a control lever (51) in whose upper side are inserted signal-emitting members (110a, 110b) by the operation of which it is possible to control functions of the apparatus and/or of peripheral units cooperating with the apparatus, so that the viewing person does not have to move his eyes away from the viewing unit (3), and also, preferably, manual adjusting activities can be carried out at the same time with the hand operating the control lever (51).

8. Apparatus according to claim 5 or 6, especially according to claim 7, **characterised by** a member which generates character information and by an optical coupling unit with which the image can be focused into at least one beam path of the viewing unit for observation with the eyepiece of the viewing unit, and the image information preferably displays data which are adjusted or changed especially by operating the control lever.

9. Apparatus according to any one of claims 1 to 8, **characterised in that** the illumination unit (5) has at least one optical filter, especially a blue and/or a grey filter, which can be introduced into the illuminating beam and, preferably, the viewing unit (3) has at least one further filter, especially a yellow filter (58), which can be introduced into the viewing beam path.

10. Apparatus according to any one of claims 1 to 9, **characterised by** a lens-carrying unit (203) which can be attached and removed again manually by means of a rotationally secure plug-in coupling (201) without using tools and which has an examination lens (204) which is then positioned in the observation beam path in front of the observation beam inlet into the viewing unit (3) and which is used especially to examine the vitreous body and the ocular fundus of the patient's eye (1), the examination lens (204) is held to be rotatable in a self-holding manner and to be adjustable in all spatial directions in a self-holding manner by the lens-carrying unit (203), the carrying unit (203) not being mechanically connected to other apparatuses, such as, for example, a head and chin holder, adjacent to the apparatus.

11. Apparatus according to claim 10 and preferably according to claim 2, **characterised in that** the coupling (201) is in the form of a rotationally secure plug-in coupling (207, 209, 210, 211) advantageously with a support member (205) for support on the holding unit (7), a coupling portion of the coupling (201) in the holding unit (7) preferably being in the form of a central axial bore (209) for the upright pivot axis (14) of the holding unit (7), an appropriate pin (207) of the lens-carrying unit (203) being in a form which can be plugged into the axial bore (209), and the rotational security preferably being formed by at least one notch member (210, 211) having a notch partial member (211) on the holding unit (7) and a further notch partial member (210) on the lens-carrying unit (203), the two partial members (210, 211) being pluggable into one another.

12. Apparatus according to either claim 10 or claim 11, **characterised by** a coupling member (207) by means of which the lens-carrying unit (203) can be placed manually, without the use of tools, on a holding unit (7) for an illumination unit (5) of an apparatus according to any one of claims 1 to 11, an examination lens (204), preferably suitable for examining the vitreous body and the ocular fundus of a patient's eye (1), a two-part lens stand (223), the one part (229) of which can be displaced towards the other (225) in a self-holding manner in order to adjust the height of the examination lens (204), and a displacement device (214, 217, 220) for displacing the lens stand (223) in two mutually perpendicular horizontal directions.

13. Apparatus according to claim 12, **characterised in that** the one part (229) of the lens stand (223) has a rod (227) which can be displaced with a clearance fit in a blind hole (226) of the other part (225) of the lens stand (223), the rod (227) preferably comprises ferromagnetic material, a permanent magnet (231) whose movement is restricted and which therefore cannot be lost is arranged in the blind hole (226), which permanent magnet (231) presses against the surface of the rod (227) which is inserted into the blind hole (226) and thus holds the one part (229) of the lens stand (223) in position once it has been adjusted.

14. Apparatus according to claim 12 or 13, **characterised by** a strip-like lens-holding member (235) which has a chain-like structure and the one end of which is held, in the longitudinal direction of the member, on the one part (229) of the lens stand (223) in a manner clampable by a resilient member (236), a lens support (232) for the examination lens (204) likewise on the one part (229) of the lens stand (223) and a suspension member (241a, 241b) for the end region of the lens-holding member (235) clampable around the lens edge (233), so that the examination lens (204) can be exchanged in a simple manner for another examination lens preferably having a different focal distance.

15. Apparatus according to any one of claims 12 to 14, **characterised by** a lens-tilting unit for tilting the examination lens (204), the tilting unit having three pivot hinges which are arranged at a distance from one another and which have mutually parallel pivot axes, so that the central point of the lens can be held at a predetermined point in space when the lens is tilted.

## Revendications

1. Dispositif, notamment appareil de lampe à fente, pour l'observation stéréoscopique d'un oeil (1) d'un patient, comportant une unité d'observation (3, 65), dans lequel l'oeil (1) peut être éclairé par un filet de lumière (9), qui sort d'une unité d'éclairement (5) et possède une section transversale prédéterminée, l'unité d'éclairement (5) est disposée en position haute sur une branche verticale (20) d'une unité de retenue (7), l'oeil (1) à observer peut être positionné dans un plan approximativement horizontal d'un côté de l'unité de retenue (7) et une unité de l'observation (3) est disposée approximativement dans le plan sur le côté, situé à l'opposé de cette unité, de l'unité de retenue (7), **caractérisé en ce que** la branche verticale (20), qui porte l'unité d'exposition (5) de l'unité de retenue (7) est agencée sous la forme d'une seule colonne possédant une section transversale étroite et sur laquelle est disposé un miroir de renvoi (10), à l'aide duquel le rayonnement (9) de l'unité d'éclairement (5) peut être dirigé dans ou sur l'oeil (1), ce qui permet d'obtenir un bon contact entre le patient et le praticien avec seulement une faible perturbation optique entre l'unité d'observation (3) et l'oeil (1) du patient.

2. Dispositif selon la revendication 1, **caractérisé en ce que** l'unité de retenue (7) est agencée en forme de L, de préférence d'un seul tenant, l'unité d'éclairement (5) étant disposée sur une extrémité du L, tandis que l'autre extrémité du L est retenue dans une articulation pivotante (15) possédant un axe vertical de pivotement (14).

3. Dispositif selon la revendication 1 ou 2, **caractérisé par** une seconde unité de retenue (23), en forme de L, agencée de préférence d'un seul tenant, pour l'unité d'éclairement (3), l'unité d'éclairement (3) étant disposée sur une extrémité du L tandis que l'autre partie d'extrémité du L est retenue dans une articulation pivotante (15) possédant un axe vertical de pivotement (14), et les axes de pivotement (14) pour les unités de retenue (7, 23) de l'unité d'éclairement (3) et de l'unité d'éclairement (5) étant convergés.

4. Dispositif selon l'une des revendications 1 à 3, **caractérisé en ce que** l'unité de retenue (7) pour l'unité d'éclairement (5) est creuse intérieurement et inclut une cavité, l'élément de transmission de force pour le réglage de la section transversale d'une ouverture de diaphragme dans l'unité d'éclairement (5) étant guidé dans la cavité pour la production d'une section transversale prédéterminée du filet de lumière, et l'élément de réglage (21a, 21b), qui agit sur l'élément de transmission de force, est disposé de préférence à l'origine des branches de la seconde unité de retenue (7) en forme de L.

5. Dispositif selon l'une des revendications 1 à 4, **caractérisé en ce que** le microscope stéréoscopique est un microscope de Greenough, qui ne possède aucun trajet de rayonnement collimaté (non parallèle) et, qu'un faisceau partiel (42b, 71a) peut être occulté à partir d'au moins un trajet du faisceau (30a, 73a) du microscope au moyen d'une unité de couplage et/ou de découplage d'image (37, 75, 76), l'information d'image de ce faisceau partiel étant envoyée de préférence à une unité de réception (46, 79) disposée dans l'unité d'observation (3, 65).

6. Dispositif selon la revendication 5, **caractérisé en ce que** l'unité de réception (79) est agencée de manière à être enfichée dans l'unité d'observation (65) et à en être extraite, et de préférence le découplage du faisceau partiel (71a) s'effectue en direction de l'unité de réception (79), par une division géométrique du faisceau.

7. Dispositif selon l'une des revendications 1 à 6, **caractérisé en ce que** pour le positionnement dans les directions X et Y (c'est-à-dire dans un plan horizontal) et de préférence dans une direction Z (réglage en hauteur) du filet de lumière (9) devant être dirigé dans l'oeil (1) du patient il est prévu, conjointement avec l'unité d'observation (3), un levier d'orientation (51), dans le côté supérieur duquel sont insérés des éléments (110a, 110b) délivrant des signaux et dont l'actionnement permet de commander des fonctions du dispositif et/ou des unités périphériques, qui coopèrent avec le dispositif, afin que la personne effectuant l'observation n'ait pas à écarter son regard de l'unité d'observation (3), et que de préférence des activités manuelles de réglage puissent être également réalisées simultanément avec la main qui actionne le levier d'orientation (51).

8. Dispositif selon la revendication 5 ou 6, notamment selon la revendication 7, **caractérisé par** un élément qui produit des informations sous forme de symboles, et une unité de couplage optique, avec laquelle l'image peut être injectée dans au moins un trajet de rayonnement de l'unité d'observation à l'aide d'une observation avec l'oculaire de l'unité d'observation, et l'information d'image indique de préférence des données qui sont réglées ou modifiées notamment par actionnement du levier d'orientation.

9. Dispositif selon l'une des revendications 1 à 8, **caractérisé en ce que** l'unité d'éclairement (5) possède au moins un filtre optique pouvant être inséré dans le trajet d'éclairement, notamment un filtre bleu et/ou un filtre gris, et de préférence l'unité d'observation (3) possède au moins un autre filtre pouvant être introduit dans le trajet du rayonnement d'observation, notamment un filtre jaune (58).

10. Dispositif selon l'une des revendications 1 à 9, **caractérisé par** une unité (203) formant porte-lentille qui peut être introduite et à nouveau retirée manuellement, sans l'utilisation d'outils, au moyen d'un accouplement à enfichage (201) bloqué contre toute rotation et qui comporte une lentille d'examen (204), qui vient alors se placer dans le trajet du rayonnement d'observation, devant l'entrée du rayonnement d'observation dans l'unité d'observation (3), et sert notamment à examiner l'humeur vitrée et le fond de l'oeil (1) du patient, la lentille d'examen (204) est retenue avec auto-maintien de manière à pouvoir tourner et avec auto-maintien de manière à être déplaçable dans toutes les directions spatiales avec l'unité (203) formant porte-lentille, l'unité (203) n'ayant aucune liaison mécanique avec d'autres dispositifs voisins, comme par exemple un appui-tête et un appui pour le menton.

11. Dispositif selon la revendication 10 et de préférence selon la revendication 2, **caractérisé en ce que** l'accouplement (201) est agencé sous la forme d'un accouplement à enfichage (207, 209, 210, 211) bloqué contre une rotation, comportant avantageusement un élément d'appui (205) destiné à s'appuyer sur l'unité de retenue (7), et dans lequel une partie de l'accouplement (201) est agencée de préférence sous la forme d'un perçage axial centré (209) pour l'axe vertical de pivotement (14) de l'unité de retenue (7), et dans lequel un axe adapté (207) de l'unité (203) formant porte-lentille est agencé de manière à être enfichable dans le perçage d'axe (209), et le blocage en rotation est formé de préférence par au moins un élément d'encliquetage (210, 211) comportant respectivement un élément partiel d'encliquetage (211) situé sur l'unité de retenue (7), et un autre élément partiel d'encliquetage (210) situé sur l'unité (203) formant porte-lentille, les deux éléments partiels (210, 211) pouvant être enfichés l'un dans l'autre.

12. Dispositif selon l'une des revendications 10 ou 11, **caractérisé par** une partie d'accouplement (217), avec laquelle l'unité (203) formant porte-lentille peut être mise en place manuellement, sans l'utilisation d'outils sur un dispositif de retenue (7) pour une unité d'observation (5) d'un dispositif selon l'une des revendications 1 à 11, une lentille d'examen (204) convenant de préférence pour examiner l'humeur vitrée et le fond d'un oeil (1) d'un patient, un second porte-lentille (223), dont une partie (229) est déplaçable, avec un auto-maintien, par rapport à l'autre partie (225) pour le réglage en hauteur de la lentille d'examen (204), ainsi qu'un dispositif de translation (214, 217, 220) servant à translater le porte-lentille (223) dans deux directions horizontales perpendiculaires entre elles.

13. Dispositif selon la revendication 12,
**caractérisé en ce qu'**une partie (229) du porte-lentille (223) possède un mandrin (227), qui peut être déplacé avec un jeu adapté dans un trou borgne (226) de l'autre partie (225) du porte-lentille (223), que le mandrin (227) est réalisé de préférence en un matériau ferromagnétique, et que dans le trou borgne (226) est disposé un aimant permanent (231), qui a une mobilité limitée et par conséquent est imperdable et qui prend appui contre la surface du mandrin (227) inséré dans le trou borne (226) et par conséquent maintient la partie (229) du porte-lentille (223) dans une position réglée une fois pour toutes.

14. Dispositif selon la revendication 12 ou 13, **caractérisé par** un élément (235) de retenue de lentille en forme de bande, qui possède une structure en forme de chaîne et dont une extrémité est retenue d'une manière à pouvoir être tendue dans une direction longitudinale de cet élément, sur une partie (229) du porte-lentille (223) comportant un élément de ressort (236), un support (232) pour la lentille d'examen (204) installée également sur une partie (229) du porte-lentille (223) et un élément d'accrochage (241a, 241b) pour la partie d'extrémité de l'élément (235) de retenue de lentille, qui peut être serré autour du bord (233) de la lentille, afin que la lentille d'examen (204) puisse être remplacée de façon simple par une autre lentille d'examen, possédant de préférence une autre distance focale.

15. Dispositif selon l'une des revendications 12 à 14, **caractérisé par** une unité de basculement de la lentille servant à faire basculer la lentille d'examen (204), l'unité de basculement comportant trois articulations pivotantes qui sont distantes les unes des autres et possèdent des axes de pivotement parallèles entre eux afin que le centre de la lentille puisse être maintenu, lors du basculement de la lentille, en un point spatial prédéterminé.
